Europäisches Patentamt

European Patent Office

Office européen des brevets

(19)

(11) Veröffentlichungsnummer: **0 038 902**
**A2**

(12) ## EUROPÄISCHE PATENTANMELDUNG

(21) Anmeldenummer: **81100522.2**

(22) Anmeldetag: **24.01.81**

(51) Int. Cl.³: **A 61 F 1/00**
**A 61 F 1/03**

(30) Priorität: **29.04.80 CH 3297/80**

(43) Veröffentlichungstag der Anmeldung:
**04.11.81 Patentblatt 81/44**

(84) Benannte Vertragsstaaten:
**AT DE FR GB IT NL**

(71) Anmelder: **GEBRÜDER SULZER AKTIENGESELLSCHAFT**
**Zürcherstrasse 9**
**CH-8401 Winterthur(CH)**

(72) Erfinder: **Frey, Otto**
**Walrütistrasse 56**
**CH-8400 Winterthur(CH)**

(74) Vertreter: **Dipl.-Ing. H. Marsch Dipl.-Ing. K. Sparing**
**Dipl.-Phys.Dr. W.H. Röhl**
**Rethelstrasse 123**
**D-4000 Düsseldorf(DE)**

(54) **Oberflächenstruktur für Verankerungselemente von Knochenimplantaten.**

(57) Mindestens Teilflächen von Verankerungselementen (1) für Knochenimplantate (2) sind mit einer flächenhaften Auflage (3) belegt, die an einer Anzahl einzelner Haftstellen (5) mit dem Grundmaterial (9) des Verankerungselementes (1) fest verbunden sind.

Die Anzahl der Haftstellen (5) ist dabei mindestens so gross, dass ihre Gesamt-Haftfestigkeit der Belastbarkeit des spongiosen Knochengewebes entspricht.

Durch die als flächenhafte Auflage (3) ausgebildete Oberflächenstruktur ist es möglich, zum einen für Auflage (3) und Grundmaterial (9) Materialien mindestens unterschiedlichen Gefüges zu verwenden; zum anderen können dadurch Aenderungen im Gefüge oder der Zusammensetzung des Grundmaterials (9), die beim Aufbringen durchgehend fest haftender Oberflächenschichten entstehen, vermieden werden.

Fig. 1

Gebrüder Sulzer, Aktiengesellschaft, Winterthur/Schweiz

Oberflächenstruktur für Verankerungselemente von Knochenimplantaten

Die Erfindung betrifft eine Oberflächenstruktur für Verankerungselemente von Knochenimplantaten, wobei mindestens auf
einem Teil der Verankerungsfläche verteilte Hohlräume vorgesehen sind.

Bekanntlich ist für eine gute Verankerung von Implantaten,
insbesondere von Gelenkendoprothesen eine möglichst grosse
Oberfläche anzustreben, durch die hindurch belastende Kräfte
von dem Implantat auf den Knochen - gegebenenfalls über ein
dazwischen liegendes vollständiges oder teilweises Knochenzementbett - übertragen werden.

Es sind daher schon eine Vielzahl von Oberflächenstrukturen
für Verankerungselemente derartiger Implantate bekannt geworden. Eine Art dieser Strukturen besteht in einzelnen
oder untereinander verbundenen Poren, die entweder im Grundmaterial der Verankerungselemente angeordnet oder durch nachträgliches Aufbringen einer zusätzlichen Oberflächenschicht
hergestellt werden (z.B. US-PS 3,855,638). In vielen Fällen
sind diese Poren in zusätzlichen Oberflächenbeschichtungen
enthalten, die korrosionsbeständig auf dem Grundmaterial haften
müssen und vorzugsweise durch Flammspritzen oder Sintern fest
mit dem Grundmaterial verbunden werden. Die geschilderten
Verfahren zur Herstellung poröser Oberflächen führen zu Eingriffen und Störungen - beispielsweise durch starke örtliche
Erwärmungen - in das "gesunde" Gefüge des Grundmaterials, das
unter Umständen durch vorherige Wärmebehandlungen in einen
möglichst störungsarmen Zustand gebracht worden ist.

Die genannten Gefügeänderungen bilden häufig Schwachstellen des Grundmaterials, von denen Korrosionen oder mechanische Zerstörungen ausgehen.

Andererseits sind diese Oberflächenstrukturen der Verankerungselemente häufig als Gewinde oder als Vorsprünge und Vertiefungen verwirklicht worden (siehe z.B. DE-PS 837 294 und 22 05 808, sowie DE-OS 29 14 513). Obwohl durch abgerundete Formgebung scharfe Ecken und Kanten vermieden werden können, die an den dabei auftretenden Uebergängen zwischen dem Kern- oder Grundmaterial der Verankerungselemente und der Oberflächenstruktur zu Rissen und daraus entstehenden Brüchen führen, haben diese Oberflächenstrukturen oftmals den Nachteil, dass sie aus dem Grundmaterial der Verankerungselemente bestehen. Sehr häufig wird jedoch die Forderung gestellt, dass die Oberflächenschicht aus einem Material mit anderer Zusammensetzung als das Grundmaterial herzustellen oder ihm - bei gleichem Material - mindestens ein abweichendes Gefüge zu geben; so kann beispielsweise gefordert werden, dass der Kern mit einem geschmiedeten Gefüge, die Oberfläche jedoch mit einem kaltverformten Gefüge, beispielsweise demjenigen eines Bleches aus Material,gleicher Zusammensetzung versehen ist.

Weiterhin ist es bekannt, Verankerungselemente mit einem grob-maschigen Netz zu überziehen, ohne dass dieses mit dem Grundmaterial des Verankerungselementes fest verbunden wird. Dieses Netz, das eine Armierung eines Zementbettes ist und dessen Festigkeit erhöhen soll, ist damit jedoch keine Oberflächenstruktur des Verankerungselementes selbst (US-PS 4,064,567).

Aufgabe der Erfindung ist es, Verankerungselemente zu schaffen, bei denen die Oberflächenschicht aus einem mindestens hinsichtlich des Gefüges vom Kern verschiedenen Material besteht, und/oder gleichzeitig die Herstellung oder das Aufbringen der Oberflächenstruktur das Grundmaterial in seinem Gefüge

- 3 -

0038902

nicht oder nur so wenig wie möglich verändern und beeinträchtigen. Die Lösung dieser Aufgabe erfolgt erfindungsgemäss dadurch, dass auf Teilbereichen des Verankerungselementes eine mit Durchbrüchen versehene, flächenhafte Auflage vorgesehen ist, die in einer Anzahl auf ihrer Fläche
verteilter Haftstellen fest an die Oberfläche des Verankerungselementes angeheftet ist, wobei die Anzahl der Haftstellen
so gewählt ist, dass die Haftfestigkeit aller Haftstellen
mindestens der Belastbarkeit des Knochens entspricht.

Die neue Oberflächenstruktur ist bei allen in der Implantat-
Technik gebräuchlichen Werkstoffen anwendbar und kann für
Verankerungselemente angewendet werden, die zementfrei,
zementarm oder über ein konventionelles Zementbett im Knochen
verankert sind.

Da sowohl die Fläche eines einzelnen Haftpunktes als auch
seine Scherfestigkeit durch Versuche einfach zu ermitteln
sind, bereitet es keine Schwierigkeiten, die notwendige Anzahl von Haftstellen bei einer gegebenen Grösse der flächenhaften Auflage festzustellen. Die Haftfestigkeit aller Haftstellen kann auf die Scherbelastbarkeit des spongiosen Knochengewebes beschränkt werden, weil diese im allgemeinen die
niedrigste Belastbarkeit in der Kette "Grundmaterial,
Auflage,- gegebenenfalls - Zementbett und Knochengewebe"
sowie der dazwischen liegenden Grenzflächen oder Uebergänge
ist.

Durch das punktweise Anheften der Auflage wird das Grundmaterial nur minimal beansprucht, so dass seine Eigenschaften
dadurch nicht verändert werden. Die flächenhaften Auflagen
können zweckmässigerweise, beispielsweise Lochplatten oder
Gewebe sein. Das Anheften erfolgt vor allem bei Metallen
und Kunststoffen vorteilhaft durch Punktschweissen; es ist
aber auch möglich, zwischen Auflage und Grundmaterial eine
Klebeverbindung herzustellen, besonders wenn ein oder beide Werkstoffe nicht gut schweissbar sind.

Es liegt auf der Hand, dass die flächenhaften Auflagen gegenüber dem Grundmaterial mindestens ein anderes Gefüge, eine
andere Zusammensetzung haben und/oder auch einer anderen
Stoffklasse angehören können. Insbesondere kann es vorteilhaft sein, eine flächenhafte Auflage aus dem besonders
körperverträglichen Titan bzw. einer Titanlegierung auf ein
- wegen der geforderten mechanischen Eigenschaften - aus
einem anderen Metall oder einer anderen Metalllegierung
bestehendes Verankerungselement aufzubringen.

Im folgenden wird die Erfindung anhand von Ausführungsbeispielen im Zusammenhang mit der Zeichnung näher erläutert.

Fig. 1 ist eine Ansicht einer Hüftgelenkprothese,
deren Schaft teilweise mit der neuen Oberflächenstruktur versehen ist.

Fig. 2 stellt in einem Ausschnitt einen Schnitt durch
ein mit einem Gewebe als Auflage versehenen
Verankerungselement dar;

Fig. 3 zeigt in gleicher Darstellung wie Fig. 2 eine
mit einer Lochplatte belegte Oberfläche.

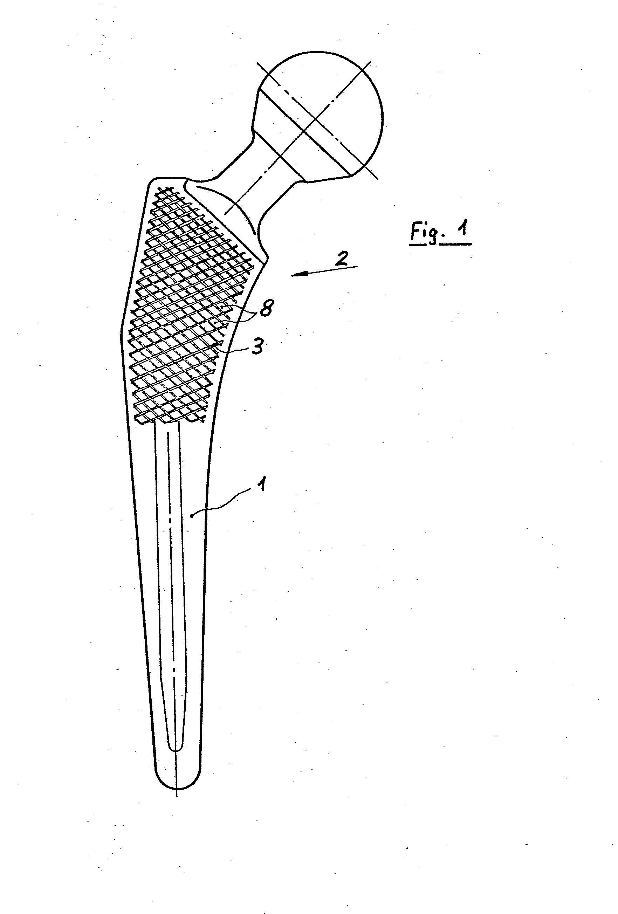

Ein blattartiger Schaft 1 (Fig. 1) einer in Ansicht in
sagittaler Richtung dargestellten Hüftgelenkprothese 2 ist
in seinem proximalen Bereich gemäss der vorliegenden Erfindung
mit einer flächenhaften Auflage 3 belegt, die im vorliegenden
Fall aus einem relativ grobmaschigen Gewebe aus einzelnen
Fäden oder Drähten 4 besteht, zwischen denen Durchbrüche 8
vorhanden sind.

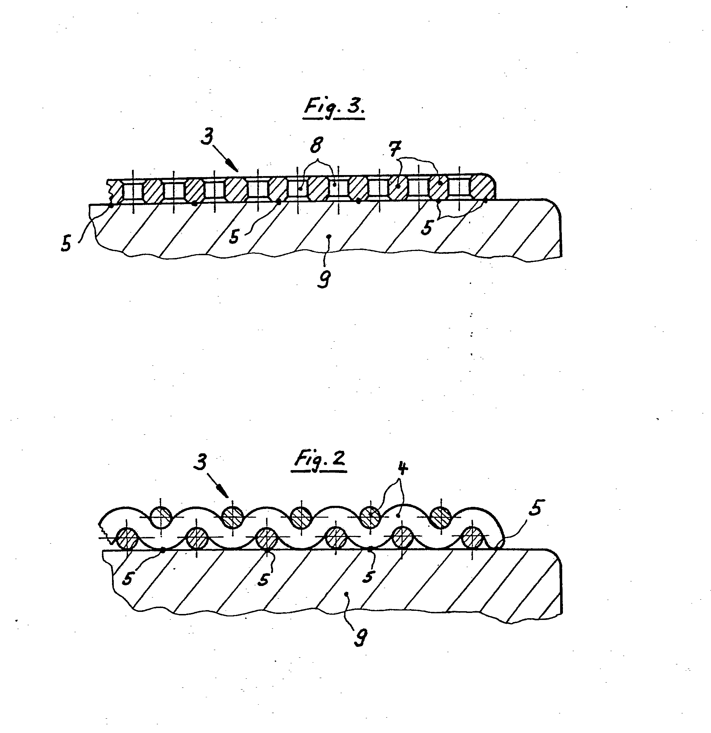

Wie Fig. 2 zeigt, ist das Gewebe auf dem Grundmaterial 9 nur
an einzelnen, in Fig. 2 und 3 durch gegenüber ihrer tatsächlichen Grösse stark übertrieben hervorgehobenen Haftstellen 5 befestigt, die bei metallischem Grundmaterial 9
und Gewebe aus Metalldrähten 4 beispielsweise durch Punktschweissen hergestellt sind.

Da es keinerlei Schwierigkeiten bereitet, die Flächengrösse der einzelnen Haftstelle 5 experimentell zu ermitteln, und auch die Scherfestigkeit einer einzelnen oder einer bekannten Anzahl derartiger Haftstellen 5 durch einfache in der Messtechnik der Materialuntersuchungen bekannte Versuche bestimmt werden kann, ist es keine Schwierigkeit, die Mindestanzahl an Haftstellen festzulegen, die pro Flächeneinheit der Auflage 3 - zur Erfüllung der Bedingung, dass die Scherfestigkeit der Gesamtheit der Haftstellen 5 grösser sein muss als diejenige des Knochens - notwendig sind.

Die in Fig. 3 gezeigte Ausführungsform der Erfindung unterscheidet sich von derjenigen nach Fig. 1 und 2 nur dadurch, dass die Auflage 3 in Fig. 3 nicht aus einem Gewebe, sondern aus einer Platte 7 besteht, in die in regelmässigen Abständen als Durchbrüche 8 Löcher eingearbeitet sind.

Um die Gefahr von Verletzungen des physiologischen Gewebes zu vermeiden, sind die Auflagen 3, sofern sie nicht die ganze Fläche des Grundmaterials bedecken, an ihren Rändern abgerundet.

Wie bereits erwähnt, können sowohl Grundmaterial als auch Auflage grundsätzlich aus allen in der Implantat-Technik üblichen Materialien bestehen; vorzugsweise ist die Erfindung jedoch für metallische Grundmaterialien geeignet, die mit einem Metall anderen Gefüges oder anderer Zusammensetzung oder einer anderen nicht metallischen Auflage belegt werden.

- 6 -     **0038902**

Patentansprüche

1. Oberflächenstruktur für Verankerungselemente von Knochenimplantaten, wobei mindestens auf einem Teil der Verankerungsfläche verteilte Hohlräume vorgesehen sind, gekennzeichnet
durch eine mit Durchbrüchen (8) versehene, flächenhafte Auflage (3) mindestens auf Teilbereichen des Verankerungselementes (1),
die in einer Anzahl auf ihrer Fläche verteilter Haftstellen (5)
fest an die Oberfläche des Verankerungselementes (1) angeheftet ist, wobei die Anzahl der Haftstellen (5) so gewählt
ist, dass die Haftfestigkeit aller Haftstellen (5) mindestens
der Belastbarkeit des Knochens entspricht.

2. Oberflächenstruktur nach Anspruch 1, dadurch gekennzeichnet,
dass die flächenhafte Auflage (3) aus einer Lochplatte (7)
besteht.

3. Oberflächenstruktur nach Anspruch 1, dadurch gekennzeichnet,
dass die flächenhafte Auflage (3) aus einem Gewebe besteht.

4. Oberflächenstruktur nach einem der vorhergehenden Ansprüche,
dadurch gekennzeichnet, dass die flächenhafte Auflage (3)
aus Titan oder einer Titanlegierung besteht.

_Fig. 1_

2

8

3

1

0038902

Fig. 3.

Fig. 2